## Europäisches Patentamt

## European Patent Office

## Office européen des brevets

(11) Publication number: **0 168 626**
**B1**

(12)

# EUROPEAN PATENT SPECIFICATION

(45) Date of publication of patent specification: **12.09.90**

(51) Int. Cl.⁵: **A 61 K 31/54,** A 61 K 31/445, A 61 L 15/16

(21) Application number: **85107124.1**

(22) Date of filing: **10.06.85**

(54) Composition for the manufacture of a medicament for the therapeutic treatment of trauma of the skin.

(30) Priority: **11.06.84 US 619274**
**13.11.84 US 670482**
**15.05.85 US 734120**

(43) Date of publication of application:
**22.01.86 Bulletin 86/04**

(45) Publication of the grant of the patent:
**12.09.90 Bulletin 90/37**

(84) Designated Contracting States:
**AT BE CH DE FR GB IT LI NL SE**

(56) References cited:
EP-A-0 091 964
WO-A-81/03421
US-A-4 141 976

CHEMICAL ABSTRACTS, vol. 98, no. 11, 14 March 1983, Columbus, OH (US); G.J.BREWER et al.: "Therapy of sickle cell anemia with membrane expander/calmodulin inhibitor classes of drugs", p. 32, no. 83369z

(73) Proprietor: **BAR-ILAN UNIVERSITY**
**P.O. Box 1530**
**IL-52 100 Ramat Gan (IL)**

(72) Inventor: **Beitner, Rivka**
**29 Ahi Dakar Street**
**Raanana (IL)**

(74) Representative: **Perani, Aurelio et al**
**c/o JACOBACCI-CASETTA & PERANI S.N.C. Via Visconti di Modrone 7**
**I-20122 Milano (IT)**

(56) References cited:
CHEMICAL ABSTRACTS, vol. 97, no. 25, 20 Dec 1982, Columbus, OH (US); J.P.LEES-MILLER et al.: "Drugs that block calmodulin activity inhibit cell-to-cell coupling in the epidermis of Tenebrio molitor", p. 100, no. 208303m

CHEMICAL ABSTRACTS, vol. 97, no. 9, 30 Aug 1983, Columbus, OH (US); S.RANGANATHAN et al.: "Effect of calcium(2+) blocking agents on the metabolism of low density lipoproteins in human skin fibroblasts", p. 48, no. 66196c

(56) References cited:

CHEMICAL ABSTRACTS, vol. 95, no. 9, 31 Aug 1981, Columbus, OH (US); F.F.VINCENZI: "Pharmacological differentiation of calmodulin receptors?", p. 81, no. 73530u

CHEMICAL ABSTRACTS, vol. 94, no. 19, 11 May 1981, Columbus, OH (US); M.VOLPI et al.: "Local anesthetics, mepacrine, and propranolol are antagonists of calmodulin", p. 37, no. 150122w

CHEMICAL ABSTRACTS, vol. 94, no. 17, 27 April 1981, Columbus, OH (US); W.G.L.KERRICK et al.: "Calcium-regulatory mechanisms. Functional classification using skinned fibers", p. 382, no. 135449q

CHEMICAL ABSTRACTS, vol. 93, no. 11, 15 Sep 1980, Columbus, OH (US); N.D.CROSBY et al.: "Effects of phenothiazines on calcium induced contractions of chemically skinned smooth muscle", p. 45, no. 106970b

CHEMICAL ABSTRACTS, vol. 101, no. 16, 15 Oct 1984, Columbus, OH (US); p. 392, no. 137093t

CHEMICAL ABSTRACTS, vol. 98, no. 23, 06 June 1983, Columbus, OH (US); E.NAITOH et al.: "Different effects of calmodulin inhibitors on ornithine decarboxylase induction by 12-O-tetradecanoylphorbol-13-acetate in mouse skin", p. 203, no. 193087p

CHEMICAL ABSTRACTS, vol. 93, no. 11, 15 Sep 1980, Columbus, OH (US); N.D.CROSBY et al.: "Effects of phenothiazines on calcium induced contractions of chemically skinned smooth muscle", p. 45, no. 106970b

CHEMICAL ABSTRACTS, vol. 101, no. 16, 15 Oct 1984, Columbus, OH (US); p. 392, no. 137093t; & JP-A-59 95 212

## Description

Background of the invention

In the prior art there is no known therapeutic agent that has been specifically used in the treatment of trauma to the skin, for example burns, sunburn and frostbite. The general prior art approach to skin trauma therapy has been limited to symptomatic treatment and the use of specific medicaments for the alleviation of pain, infection and electrolyte imbalances. Various types of bandages have been used as protective layers for the affected areas of the body and these have contained different anesthetic and anti-infective agents.

The applicant has discovered that a certain class of chemical substances is capable of exerting a profound effect on traumatized tissue, particularly burns, sunburn and frostbite. These compounds promote healing of skin tissue and aid in preventing or alleviating the damaging effects of trauma on the skin of the mammalian body. These chemical substances possess the ability to interfere with the action of the calcium calmodulin complex in vivo in the mammalian body. Compounds which are able to bind the protein calmodulin in vivo are known in the prior art. For example, U.S. 4,443,446 describes these compounds and their use as vaginal contraceptives. Many of these compounds have also been used for their antipsychotic action.

Compounds that may be utilized for the purpose of the present invention include those phenothiazines, thioxanthenes, butyrophenones, diphenylbutylamines, dibenzodiazepines, benzodiazepines, dibenzazepines and naphthalenesulfonamides, which have the aforesaid ability to interfere with calcium calmodulin activity. Accordingly it is a primary object of this invention to provide a composition for the manufacture of a medicament for treating trauma to the skin. This object is achieved by a composition according to the appended Claim 1. The features of the invention will become apparent from the detailed description which follow.

Brief description of the drawings

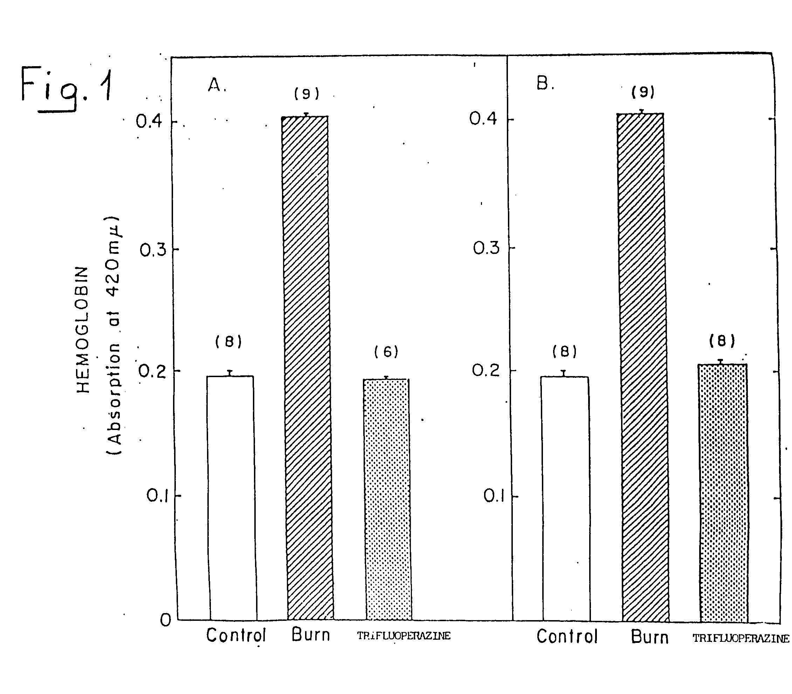

Fig. 1 is a graph that shows the reduction of hemoglobin in the skin of an animal treated with a compound according to the invention.

Detailed description of the invention

The invention relates to the use of a composition comprising an ointment, cream or lotion which contains from 4% to 40% by weight of a compound that has the ability to interfere with the action of calcium calmodulin complex in the preparation of a medicament for the therapeutic treatment of trauma to the skin.

More particularly, said medicament is useful for the treatment of mammalian tissues that have been damaged by trauma induced by thermal, electrical, radiant, freezing or mechanical stresses.

As used herein and in the appended claims, the term "compound having the ability to interfere with action of the calcium calmodulin complex" means all compounds that inhibit the in vivo actions of calcium-calmodulin complex sufficiently so that a trauma to mammalian skin will be prevented or alleviated. In particular the trauma may include a burn, sunburn or frostbite. These compounds include compounds known in the literature as anti-calmodulin compounds, calmodulin antagonists and calmodulin binding compounds. These various mechanisms that are involved are described in: Vincenzi, F. F., in Calmodulin and Intracellular Ca$^{++}$ Receptors, (S. Kakiuchi et al editors), Plenum Press, NY (1982) pp. 1—17, which is incorporated by reference.

Compounds which are known to have ability to interfere the action of calcium calmodulin complex include trifluoperazine, fluphenazine, thioridazine, chlorprothixene chlorpromazine, penfluridol, benperidol, haloperidol, pimozide, clozapine, medazepam, chlordiazepoxide, imipramine, amitriptyline, protriptyline, desipramine and naphthalenesulfonamides. The compositions for use in the preparation of the medicament of the present invention comprise:

sterile and non-sterile topical ointments, creams or lotions which contain a compound having the ability to interfere with calcium calmodulin complex; sterile and non-sterile solutions for spray application from pressurized or non-pressurized containers which contain a compound having the ability to interfere with the action of calcium calmodulin complex with or without a local anesthetic; an electrolyte solution which also contains a compound having the ability to interfere with the action of calmodulin complex; a bandage dressing which contains a compound having the ability to interfere with the action of calcium calmodulin complex; a composition which contains an anti-infective compound and said compound and a composition which contains an analgesic and said compound; a solution, for use with disposable injectable syringes, which contain a compound having the ability to interfere with the action of calcium calmodulin complex with or without an analgesic; a sterile or non-sterile ointment, cream or lotion, for use with collapsible tubes, based on a compound having the ability to interfere with the action of calcium calmodulin complex with or without an anti-infective agent and/or a local anesthetic agent; compositions for treatment of burns on the buccal mucosa or the esophogous which contain a thixotropic agent, a local anesthetic and a compound having the ability to interfere with the action of calcium calmodulin complex. Wax or stiff paste like compositions that are applied to the skin as frostbite preventatives or burn protective coatings are also within the scope of the present invention.

These compositions include lip coatings, nose coatings which contain a compound having the ability to interfere with the action of calcium calmodulin complex for application to the skin prior to exposure to low temperatures.

Compositions for the prevention of sunburn which include a sunscreen such as para-aminobenzoic acid may be combined with a compound having the ability to interfere with the action of calcium calmodulin complex to protect those who expect to be exposed to the harmful effects of the sun.

The topical ointment, cream or lotions may be based on any of the well known ointment bases, creams or lotions that are described in the literature or which are commercially available.

Suitable bases are described in Remington's Practice of Pharmacy 9th Edition, Cook et al., pp. 286—296, Mack Pub. Co. which is incorporated by reference. The concentration of the compound having the ability to interfere with the action of calcium calmodulin complex in the topical ointment cream or lotion may be any amount that is effective for treatment of burns, sunburn or frostbite or other trauma. These amounts are preferably between 1 and 50% or more preferably from 4% to 40% and most preferably 7.5 to 20% weight by total weight of composition. These compositions are preferably sterile so that they will not introduce any pathogenic organisms into a burn.

Solutions for spray applications may be for using any liquid vehicle that will provide a homogeneous dispersion of the active compounds. It is preferred to use a lipophilic vehicle that will adhere to the skin and facilitate percutaneous absorption of the active compounds. Suitable vehicles include fatty acids that are liquid at room temperature, liquid glycols, DMSO and the like. If necessary, pharmaceutically acceptable organic solvents may be utilized to dissolve or disperse the active compound. The compound may be preferred at from 1 to 50% preferably 4% to 50% and most preferably 7-1/2 to 20% by weight. These solutions may be supplied in pressurized containers using any suitable inert gas as a propellant. The solution in the container should be sterile. A non-pressurized spray container using conventional piston type applicators may also be utilized.

It is also contemplated that a suitable local anesthetic may be combined with the spray solution or with any topical preparation to provide immediate relief from the burn pain. These local anesthetics may be included at from 0.1 to 10% by weight of solutions depending on the particular local anesthetic. The useful local anesthetic include lidocaine hydrochloride, benzocaine and the like. Other local anesthetics are described in Text Books of Organic Medicinal and Pharmaceutical Chemistry, 3rd edition, Wilson et al., Lippincott Pub., 1956, pp. 421—435, which is incorporated by reference.

Electrolyte solution containing conventional quantities of electrolyte may be combined with a compound having the ability to interfere with the action of calcium calmodulin complex. Examples of these solutions include Ringer's Lactate solution and Isotonic saline. These solutions may contain from 0.1 to 10 mg per liter of the active compounds. A bandage dressing may be made from a combination of an absorbent layer that is provided with a impervious backing material. The material is saturated with a solution containing from 1 to 50% preferably 4% to 40% and most preferably 7.5% to 20% by weight of the active compound. The back layer may be made from a Mylar film or an aluminum foil layer. The absorbent layer may be made from gauze or a woven cotton fabric. An additional layer may be used to prevent it from adhering to the skin.

Compositions may be made containing an anti-infective compound solution as a bacteriostatic, bacteriocidal, antibiotic or a fungicidal agent. The particular anti-infective compound should be used at a level that will prevent the growth of pathogenic organisms. These materials are well known and include quaternary ammonium salts, neomycin, bacitracin, polymyxin, amphotericin, silver sulfadiazine, mafenide acetate, cerium sulfadiazine, gentamicin or silver nitrate and the like. These materials may be used in conventional amounts such as are shown in the Physicians Desk Reference (PDR) 38th Edition which is incorporated by reference. The amount of the compounds having the ability to bind calmodulin will vary from as low as 0.1 to 50% usually from 1 to 50% may be used, preferably 4% to 40%; and most preferably 7.5% to 20% by weight.

Disposable syringes may be prefilled with solutions of the active compounds with or without an analgesic compound such as meperidene, morphine, pentazocine and alike. The amount of the analgesic may be within the conventionally used range as shown in the PDR. The amount of the active compounds may be within the range of the single dosage unit set forth herein.

Collapsible tubes fabricated from plastic or suitable metals may be prefilled with ointment, cream, lotion that contain active compounds in an amount by 1 to 50% preferably 4% to 40% and most preferably 7.5% to 20% by weight. In addition, these containers are preferably sterile and may also contain one of the anti-infective agents set forth above and a local anesthetic.

The active compounds may be added to conventional compositions used for skin rashes such as diaper rashes. These compositions include baby powder, baby lotion and ointments used for diaper rashes. In addition the active compounds may be used in the treatment of bed sores, skin ulcers, and the healing of cuts, bruises and surgical incisions by selecting an appropriate composition.

The therapeutic treatment of burns comprises the parenteral, oral or local administration of a medicament comprising an amount of the active compound that is effective to alleviate the symptoms of a burn.

Generally, the parenteral dosage will vary depending on the host. The particular compound trifluorperazine has been utilized successfully in rats at levels of 80 mg/kg body weight administered as a single dose. Other dosage regimens may be used which comprise administering the compound in divided doses over a period of days depending on the severity of the burn, the size of the involved area and the host. Generally, for humans the parenteral dose may range from 1—25 mg or more given as a single dose or larger amounts may be given in divided doses. The mode of injection may be intravenous, subcutaneous, intramuscular or otherwise. The dose will depend on the particular compound chosen and may be adjusted to give the optimum response in a given subject. Administration may also be by means of slow intravenous drip with or without the administration of anti-infective agents or electrolytes usually given to severely burned patients. The compounds may also be administered orally using tablets, capsules, suspension or aqueous solutions.

The medicaments of the present invention may be prepared using conventional techniques.

The particular compounds that may be used are selected using the procedure set forth by Levin et al. Mol. Pharmacol 12, 581—589 (1976) which is incorporated by reference. While Levin et al use the term "activator", it is now recognized that this material is the protein known as calmodulin. See Kakiuchi et al, *supra* pp. 1—3. The criteria for the inclusion of a particular compound as one having the ability to interfere with the action of calcium calmodulin complex is that the compound prevent or alleviate a trauma to mammalian skin and that it have an $I_{50}$ (activated) value of less than 320 µM in the Levin, et al., *supra* phosphodiesterase inhibition test. Especially preferred are those compounds with an $I_{50}$ (activated) value of less than 100 µM. Many phenothiazines, thioxanthenes, butyrophenones, diphenylbutylamines, benzodiazepines, dibenzodiazepines, dibenzazepines and naphthalenesulfonamides that interfere with the action of calcium calmodulin complex are commercially available. A number of these compounds are described in Drills Pharmacology in Medicine, Di Palma, 4th Edition, pp. 466—501 which is incorporated by reference and naphthalenesulfonamides are described in Hidaka, H. and Tanaka, T., Calmodulin and Intracellular $Ca^{++}$ receptors, (S. Kakuchi, et al, editors) Plenum Press, NY (1982) pp. 19—23, which is incorporated by reference. The presently preferred compound is trifluoperazine.

The applicant does not wish to be bound by any theory by which the invention operates but it is believed that the therapeutic action is based on the fact that in skin burns and other skin injuries, there is a rapid release of bradykinin and serotonin. Both of these hormones tend to increase the intracellular concentration of calcium ions which bind the protein calmodulin to form a calcium calmodulin complex. This complex activates phospholipase $A_2$. The activated phospholipase $A_2$ acts on the cell membrane phospholipids causing the destruction of the cell membrane. This causes the loss of proteins, enzymes and other cell components, and the loss of the integrity of the integumentary system as a barrier to invasion by pathogenic organisms. The calcium calmodulin complex also activates glucose 1,6-bisphosphatase which is the enzyme that degrades glucose-1,6-bisphosphate which is involved with carbohydrate metabolism. The decrease in the concentration of glucose-1,6-bisphosphate causes a decrease in glycolysis with the resulting decrease in adenosine tri-phosphate (ATP) concentration. The decrease in ATP concentration also contributes to the destruction of the cell membrane and the discharge of the cell contents as noted above. The calmodulin binding agents are believed to bind to the calcium-calmodulin complex and render it substantially biologically inert with regard to the activation of phospholipase $A_2$ and glucose-1,6-bisphosphatase.

Description of the preferred embodiments

The following studies are illustrative of the invention and are not to be construed as a limitation on the scope of the invention.

The preferred compound, trifluorperazine, was utilized to heal burns, sunburns and frostbites. Burns were induced by pouring water at 100°C on the skin of rats. The compound has a clearly visible effect on the skin in that it markedly reduced the redness of the skin after burning. Its effect was further investigated by measuring several biochemical parameters which characterize burns, sunburns and frostbites. The following parameters were measured:

(a) *Hemoglobin*: In the inflammatory response to thermal injury, increased capillary permeability is an early and important vascular event. Thermal injury causes the extravascular accumulation of erythrocytes (Green, K. L., Br. J. Exp. Pathol. 59: 38—47 (1978), and hemoglobin is released from the heat-altered red cells ("Leading Articles", Lancet 1: 153—155 (1960).

(b) *ATP*: A characteristic change of an injured tissue is a decrease in the concentration of ATP, namely, the cell energy.

(c) Enzyme's activities: In an injured tissue, due to the pathological changes in cell membrane, there is a loss from the cell of several enzymes. The activities of soluble 6-phosphogluconate dehydrogenase and particulate and soluble hexokinase were found to be markedly reduced in the burned skin.

(d) *Protein*: The concentration of protein in the burned skin is significantly decreased.

These experiments and their results will now be described in detail:

Methods

Preparation and treatment of rat skins

Charles River albino rats, aged 8—10 days (15—22 g b.wt) fed *ad libitum* were used. Skin burns were induced in the rats under anesthesia by pouring water at 100°C on the abdomen and chest area. Trifluoperazine was administered either prior to or after the induction of burns. Identical rats with and without burns served as controls. The skin from the abdomen and chest was removed and rapidly frozen between a pair of aluminium tongs precooled in liquid $N_2$. The subcutaneous fat was scraped off and discarded, and the frozen skins were stored in liquid $N_2$ until used (24 h). The frozen skins were powdered in a mortar cooled liquid $N_2$ and the powder was used for extraction of ATP, hemoglobin, protein and enzymes.

Injection

(a) Injection prior to inducing burns

The animals were injected intraperitoneally with trifluoperazine dihydrochloride (80 mg/kg body wt) in 0.1 ml saline. One hundred minutes later skin burns were induced (at 100°C). After an additional 20 min, the skin was removed and the biochemical parameters were assayed.

(b) Injection after burning

The animals were injected immediately after burning with the same solution as in (a) and the effects were studied 1 hour later.

Cream

400 mg of trifluoperazine dihydrochloride was mixed with 5 g of "Oil of Olay Beauty Fluid" (Olay Company Inc.) and applied topically.

Extraction and determination of ATP

The frozen skin powder (70—90 mg) was placed on top of 1 ml frozen 5% perchloric acid containing 1 mM EDTA, and extracted at $-10°C$. After centrifugation, the extract was neutralized with KOH. The precipitated potassium perchlorate was removed by centrifugation and the clear supernatant was used for determination of ATP. ATP was measured as described by Lowry et al. (Lowry, O. H., Passonneau, J. V., Hasselberger, F. X. and Schulz, D. W., J. Biol. Chem. 239: 18—30 (1964).

Preparation and assay for hemoglobin and enzymes

The mitochondrial and soluble fractions were separated, and hexokinase and 6-phosphogluconate dehydrogenase activities were assayed as described by Beitner et al. (Beitner, R., Lilling, G., Frucht, H., Ben-Porat, H., and Sofer, Y., Biochem. Med. 30: 369—380 (1983). 100 µl of the soluble fraction was added to 1 ml of 0.25 M sucrose and hemoglobin was estimated spectrophotometrically at 420 mµ.

Protein determination

Protein was measured by the method of Lowry et al. (Lowry, O. H., Rosenbrough, N. J., Farr, A. L. and Randall, R. J., J. Biol. Chem. 193:265—275 (1951) with crystalline bovine serum albumin as a standard.

Fig. 1 is a diagram showing the reversal of the effect of burns on hemoglobin content in rat skin by injection of trifluoperazine.

The effect of burns on hemoglobin contents in rat skin with and without trifluorperazine was demonstrated by injection of trifluoperazine prior to and after the induction of burns. Hemoglobin was extracted and assayed as described above in Methods and the results are shown in the attached Fig. 1 in which part A shows the results where trifluoperazine was injected prior to the induction of burns and part B the results where the injection was after the induction of burns. Values are means $\pm$SE with the number of experiments in parentheses, P values (burns vs. control or trifluoperazine vs. burns) are smaller than 0.005.

The control in each case was unburnt and untreated skin. "Burn" means burnt and untreated skin and "Trif" means treatment with trifluoperazine as specified.

The reversal of the effect of burns on ATP concentration in rat skins by injection of trifluoperazine prior to the induction of burns (A) or after burning (B) was demonstrated and the results are shown in the following Table I:

TABLE 1

| Conditions | ATP (µmoles/kg wet wt) | |
|---|---|---|
| | A | B |
| Control | 1211±43 (12) | 1211±43 (12) |
| Burn | 391±38 (11) | 415±39 (12) |
| Trifluoperazine | 1071±165 (6) | 937±130 (6) |

ATP was extracted and assayed as described in Methods. Values are means $\pm$S.E., with number of experiments in parentheses. P values (burns vs. control or trifluoperazine vs. burn) 0.005.

# EP 0 168 626 B1

The reversal of the effect of burns on the activity of soluble 6-phosphogluconate dehydrogenase in rat skin by injection of trifluoperazine prior to induction of burns (A) and after burning (B) was demonstrated and the results are shown in the following Table 2:

TABLE 2

| Conditions | 6-Phosphogluconate dehydrogenase activity (munit/mg protein) | |
|---|---|---|
| | A | B |
| Control | 13.86±0.42 (16) | 13.86±0.42 (16) |
| Burn | 7.04±0.23 (11) | 6.96±0.22 (13) |
| Trifluoperazine | 11.89±0.55 (14) | 10.32±0.34 (11) |

Enzyme was extracted and assayed as described in Methods. Values are means ±S.E., with number of experiment in parentheses. P values (burn vs. control or trifluoperazine vs. burn) 0.005.

The reversal of the effect of burns on the activity of soluble and mitochondrial hexokinase in rat skin by injection or trifluoperazine prior to inducing burns (A) and after burning (B) was demonstrated and the results are shown in the following Table 3:

TABLE 3

| Conditions | Mitochondrial hexokinase activity (munit/mg protein) | | Soluble hexokinase activity (munit/mg protein) | |
|---|---|---|---|---|
| | A | B | A | B |
| Control | 2.38±0.19 (8) | 2.38±0.19 (8) | 1.57±0.07 (8) | 1.57±0.07 (8) |
| Burn | 0.86±0.05 (6) | 0.85±0.04 (6) | 0.60±0.05 (6) | 0.55±0.04 (6) |
| Trifluoperazine | 1.52±0.12 (6) | 1.40±0.14 (6) | 1.02±0.05 (6) | 0.99±0.05 (6) |

Values are means ±S.E., with number of experiments in parentheses. P values (burn vs. control or trifluoperazine vs. burn) 0.005.

The reversal of the effect of burns on protein concentration in rat skin by injection of trifluoperazine prior to the induction of burns (A) or after burning (B) was demonstrated and the results are shown in the following Table 4:

TABLE 4

| Conditions | Protein (g/kg wet wt) | |
|---|---|---|
| | A | B |
| Control | 3.940±0.304 (8) | 3.940±0.304 (8) |
| Burn | 2.254±0.100 (6) | 2.543±0.172 (6) |
| Trifluoperazine | 3.419±0.284 (6) | 3.952±0.373 (6) |

Protein was measured in the soluble fraction, as described in Methods. Values are means ±S.E., with number of experiments in parentheses. P values (burn vs. control or trifluorperazine vs. burn) 0.005.

7

The reversal of the effect of burns on hemoglobin content in rat skin by trifluoperazine administered topically in the form of a cream was demonstrated and the results are shown in the following Table 5:

TABLE 5

| Conditions | Hemoglobin (absorption at 420 mμ) |
|---|---|
| Control | 0.18±0.02 (8) |
| Burn | 0.46±0.03 (10) |
| Trifluoperazine | 0.25±0.02 (10) |

Trifluoperazine was administered topically in the form of a cream, immediately following the induction of burns (at 100°C), and reapplied 1 hr later (total of two applications). After an additional hr (i.e., 2 hr from onset of burns), the skin was removed and hemoglobin extracted and assayed, as described in Methods. Values are means ±S.E., with number of experiments in parentheses. P values (burns vs. control or trifluorperazine vs. burn) 0.005.

Reversal of the effect of burns on ATP concentration in rat skin by trifluoperazine administered topically in the form of a cream was demonstrated and the results are shown in the following Table 6:

TABLE 6

| Conditions | ATP (μmoles/kg wet wt) |
|---|---|
| Control | 1308±88 (5) |
| Burn | 368±56 (5) |
| Trifluoperazine | 750±650 (5) |

Trifluoperazine was administered topically, as described in Table 5. ATP was extracted and assayed, as described in Methods. Values are means ±S.E., with number of experiments in parentheses. P Values (burn vs. control or trifluoperazine vs. burn) 0.005.

TABLE 6a
Reversal of the effect of burns on ATP concentration
in rat skin by trifluoperazine administered
topically in the form of a cream

| Conditions | ATP (μmoles/kg wet wt) |
|---|---|
| Control | 1052±34 (6) |
| Burn | 382±54 (6) |
| Trifluoperazine | 1033±86 (6) |

Trifluoperazine was administered topically in the form of a cream, immediately following the induction of burns, and reapplied 30 minutes later, and then every hour (total of seven applications). After $5\frac{1}{2}$ hours from onset of burns, the skin was removed from each animal and ATP extracted and assayed, as described in Methods. Values are means ±S.E., with number of experiments in parentheses. P values (burn vs. control or trifluoperazine vs. burn) 0.005.

Reversal of the effect of burns on the activity of soluble 6-phosphogluconate dehydrogenase in rat skin by trifluoperazine administered topically in the form of a cream was demonstrated and the results are shown in the following Table 7:

TABLE 7

| Conditions | 6-Phosphogluconate dehydrogenase activity (munit/mg protein) |
|---|---|
| Control | 16.30±0.59 (7) |
| Burn | 7.30±0.24 (15) |
| Trifluoperazine | 12.49±0.44 (11) |

Trifluoperazine was administered topically, as described in Table 5. Enzyme was extracted and assayed, as described in Methods. Values are means ±S.E., with number of experiments in parentheses. P values (burns vs. control or trifluoperazine vs. burn) 0.005.

The reversal of the effect of burns on the activity of soluble and mitochondrial hexokinase in rat skin by trifluoperazine administered topically in the form of a cream was demonstrated and the results are shown in the following Table 8:

TABLE 8

| Conditions | Mitochondrial hexokinase activity (munit/mg protein) | Soluble hexokinase activity (munit/mg protein) |
|---|---|---|
| Control | 2.33±0.23 (6) | 1.77±0.18 (6) |
| Burn | 0.88±0.05 (6) | 0.69±0.02 (6) |
| Trifluoperazine | 1.73±0.12 (7) | 1.15±0.09 (7) |

Trifluoperazine was administered topically, as described in Table 5. Hexokinase was extracted and assayed, as described in Methods. Values are means ±S.E., with number of experiments in parentheses. P values (burn vs. control or trifluoperazine vs. burn) 0.005.

Reversal of the effect of burns on protein concentration in rat skin by trifluoperazine administered topically in the form of a cream was demonstrated and the results are shown in the following Table 9:

TABLE 9

| Conditions | Protein (g/kg wet wt) |
|---|---|
| Control | 4.160±0.177 (6) |
| Burn | 2.646±0.067 (6) |
| Trifluoperazine | 3.517±0.076 (7) |

Trifluoperazine was administered topically, as described in Table 5. Protein was measured in the soluble fraction, as described in Methods. Values are means ±S.E., with number of experiments in parentheses.

P values (burn vs. control or trifluoperazine vs. burn) 0.005.

Results

(a) Effects or trifluoperazine administered as an injectible solution

When trifluoperazine was injected 100 min prior to inducing burns, it completely prevented the increase in hemoglobin content, and its concentration was similar to that of normal control skins. Similar results were obtained when trifluorperazine was injected immediately after burning and its effect was studied 1 hr later. Treatment with trifluoperazine completely reversed the effect of burning on hemoglobin content.

9

# EP 0 168 626 B1

The experiments demonstrated in Table 1 show the effect of trifluorperazine on ATP concentration in skin. It can be seen that burning induced a marked decrease in the levels of ATP in skin, whereas injection of trifluoperazine 100 min prior to inducing burns (A) almost completely prevented the decrease in the concentration of ATP. Similar results were obtained when trifluoperazine was injected after burning and its effects studies 1 hour later (B).

As shown in Table 2, under identical conditions, trifluoperazine also antagonized the effect of burns on the activity of soluble 6-phosphogluconate dehydrogenase. The activity of this enzyme, which is markedly reduced by burns, was almost completely restored by injection of trifluoperazine either prior to inducing burns (A) or after burning (B). Similar results were obtained for hexokinase (Table 3). Burns induced a marked decrease in the activity of this enzyme from both the mitochondrial and soluble fractions and trifluoperazine antagonized this effect when injected either prior to inducing burns (A) or after burning (B).

The results presented in Table 4 show the effect of trifluoperazine on protein concentration in the soluble fraction from rat skin. It can be seen that burning induced a significant decrease in protein concentration, whereas injection of trifluoperazine prior to inducing burns (A), or after burning (B), reversed this effect.

(b) Effects of trifluoperazine administered topically in the form of a cream

The results presented in Tables 5—9 show the effects of trifluoperazine administered topically to the burned skin in the form of a cream. It can be seen that the effects of trifluoperazine applied in the form of a cream for 2 hours from onset of burns were similar to those obtained by the injectable solution. Namely, the cream reversed the changes in hemoglobin, ATP and protein concentration induced by burns (Tables 5, 6 and 9 respectively).

It is also reversed the reduction in enzymes' activities in the burned skin (Tables 7 and 8).

These results show that trifluoperazine is very effective in the treatment and prevention of burns. It prevented and abolished the marked changes in the skin content of hemoglobin, ATP, protein and enzymes' activities induced by burns. It also has a clearly visible effect on the skin.

Similar results are obtained in the treatment of sunburn and frostbite.

Since the compound acts both by treating and preventing burns, sunburns and frostbite, it may be used both in first aid and burn treatment and also to protect persons exposed to fire or the sun in the course of their daily work (e.g. soldiers, firemen, outdoor workers, etc.) and as protective measure in those plants and factories where employees work with fire.

It has been found that trifluoperazine is effective in the treatment of frostbite injuries. Frostbite was induced in rats under anesthesia by applying dry ice to the skin in the abdomen area. Trifluoperazine was administered topically to the frost-injured skin in the form of 20% by weight dispersion in "Oil of Olay Beauty Fluid" (Olay Company Inc). The results as reported in Tables 10—13 show the effects on hemoglobin, ATP, and enzymes' activities in the frost injured skin. It can be seen that the changes in these biochemical parameters induced by frostbite are similar to those induced by burns, namely, and increase in hemoglobin concentration and a decrease in ATP, and enzymes' activities. These experiments reveal that trifluoperazine reverses the changes in all these biochemical parameters to normal control levels.

TABLE 10
Effect of frostbite on hemoglobin content in rat
skin and its reversal by trifluoperazine administered
topically in the form of a cream

| Conditions | Hemoglobin (absorption at 420 mμ) |
|---|---|
| Control | 0.14±0.001 (7) |
| Frostbite | 0.32±0.030 (11) |
| Trifluoperazine | 0.16±0.001 (20) |

Frostbite was induced in the rats under anesthesia by applying dry ice for 1 minute to the skin in the abdomen area. Trifluoperazine was administered topically in the form of a cream, immediately following the induction of frostbite, and reapplied 30 minutes later, and then every hour. After 5-1/2 hours from the induction of frostbite, a section of the affected skin was surgically removed and hemoglobin extracted and

10

assayed, as described supra. Values are means ±S.E., with number of experiments in parentheses. P values (frostbite vs. control or trifluoperazine vs. frostbite) 0.005.

TABLE 11

Effect of frostbite on ATP concentration in rat skin
and its reversal by trifluoperazine administered
topically in the form of a cream

| Conditions | ATP (μmoles/kg wet wt) |
|---|---|
| Control | 1087±63 (8) |
| Frostbite | 348±48 (8) |
| Trifluoperazine | 979±59 (14) |

Trifluoperazine was administered topically, as described in Table 10. ATP was extracted and assayed, as described in Methods. Values are means ±S.E., with number of experiments in parentheses. P values (frostbite vs. control or trifluoperazine vs. frostbite)<0.005.

TABLE 12

Effect of frostbite on the activity of soluble 6-phosphogluconate
dehydrogenase in rat skin and its reversal by trifluoperazine
administered topically in the form of a cream

| Conditions | 6-Phosphogluconate dehydrogenase activity (munit/mg protein) |
|---|---|
| Control | 17.73±0.59 (6) |
| Frostbite | 5.67±0.24 (6) |
| Trifluoperazine | 15.45±0.41 (6) |

Trifluoperazine as administered topically, as described in Table 10. Enzyme was extracted an assayed, as described in Methods. Values are means ±S.E., with number of experiments in parentheses. P value (frostbite vs. control or trifluoperazine vs. frostbite) <0.005.

TABLE 13

Effect of frostbite on the activity of soluble and mitochondrial
hexokinase in rat skin and its reversal by trifluoperazine
administered topically in the form of a cream

| Conditions | Mitochondrial hexokinase activity (munit/mg protein) | Soluble hexokinase activity (munit/mg protein) |
|---|---|---|
| Control | 2.93±0.11 (6) | 2.09±0.07 (6) |
| Frostbite | 1.65±0.07 (6) | 1.00±0.05 (6) |
| Trifluoperazine | 3.22±0.17 (6) | 1.99±0.08 (6) |

Trifluoperazine was administered topically, as described in Table 10. Hexokinase was extracted and assayed, as described in Methods. Values are means ±S.E., with number of experiments in parentheses. P values (frostbites vs. control or trifluoperazine vs. frostbite)<0.005.

Trifluoperazine was also found to be effective in the prevention and treatment of sunburn. Sunburn was induced in rats under anesthesia by exposing their skin in the abdomen area to a UV lamp (Osram-Sonnenstrahler Ultra-Vitalux[R]), which closely resembles natural sunlight. The distance between lamp and the animal was 17.5 cm and the time of exposure was 1-1/2—2 minutes. Trifluoperazine was administered either as an injectable solution (2 mg per rat) or topically in the form of a cream (8% of trifluoperazine in Oil of Olay).

Tables 14—21 show the effects of trifluoperazine on hemoglobin, ATP, and enzymes activity in the sunburned skin. It can be seen that trifluoperazine administered either as an injectable solution or topically in the form of a cream, reversed the changes in these biochemical parameters.

These results show that trifluoperazine has utility in the treatment of sunburn.

TABLE 14
Effect of sunburn on hemoglobin content in rat skin
and its reversal by trifluoperazine administered
as in injectable solution

| Conditions | Hemoglobin (absorption at 420 mµ) |
|---|---|
| Control | 0.16±0.015 (18) |
| Sunburn | 0.56±0.025 (15) |
| Trifluoperazine | 0.15±0.010 (14) |

Values are means ±S.E., with number of experiments in parentheses. P values (sunburn vs. control or trifluoperazine vs. sunburn) 0.005.

TABLE 15
Effect of sunburn on ATP concentration in rat skin
and its reversal by trifluoperazine administered
as an injectable solution

| Conditions | ATP (µmoles/kg wet wt) |
|---|---|
| Control | 1136±65 (10) |
| Sunburn | 393±30 (9) |
| Trifluoperazine | 922±52 (12) |

Values are means ±S.E., with number of experiments in parentheses. P values (sunburn vs. control or trifluoperazine vs. sunburn)<0.005.

TABLE 16
Effect of sunburn on the activity of soluble 6-phosphogluconate
dehydrogenase in rat skin and its reversal by trifluoperazine
administered as an injectable solution

| Conditions | 6-Phosphogluconate dehydrogenase activity (munit/mg protein) |
|---|---|
| Control | 21.26±0.47 (10) |
| Sunburn | 14.33±0.47 (18) |
| Trifluoperazine | 21.13±0.54 (15) |

Values are means ±S.E., with number of experiments in parentheses. P values (sunburn vs. control or trifluoperazine vs. sunburn)<0.005.

12

TABLE 17
Effect of sunburn on the activity of soluble and mitochondrial
hexokinase in rat skin and its reversal by trifluoperazine
administered as an injectable solution

| Conditions | Mitochondrial hexokinase activity (munit/mg protein) | Soluble hexokinase activity (munit/mg protein) |
|---|---|---|
| Control | 3.40±0.17 (9) | 2.33±0.09 (9) |
| Sunburn | 1.13±0.08 (9) | 1.29±0.07 (9) |
| Trifluoperazine | 2.54±0.19 (9) | 2.07±0.11 (9) |

Values are means ±S.E., with number of experiments in parentheses. P values (sunburn vs. control or trifluoperazine vs. sunburn)<0.005.

TABLE 18
Effect of sunburn on hemoglobin content in rat skin
and its reversal by trifluoperazine administered
in the form of a cream

| Conditions | Hemoglobin (absorption at 420 mμ) |
|---|---|
| Control | 0.16±0.015 (18) |
| Sunburn | 0.50±0.037 (7) |
| Trifluoperazine | 0.21±0.019 (12) |

Values are means ±S.E., with number of experiments in parentheses. P values (sunburn vs. control or trifluoperazine vs. sunburn)<0.005.

TABLE 19
Effect of sunburn on ATP concentration in rat skin
and its reversal by trifluoperazine administered
topically in the form of a cream

| Conditions | ATP (μmoles/kg wet wt) |
|---|---|
| Control | 1135±64 (10) |
| Sunburn | 476±68 (5) |
| Trifluoperazine | 1297±46 (9) |

Values are means ±S.E., with number of experiments in parentheses. P values (sunburn vs. control or trifluoperazine vs. sunburn)<0.005.

### TABLE 20
Effect of sunburn on the activity of soluble 6-phosphogluconate
dehydrogenase in rat skin and its reversal by trifluoperazine
administered topically in the form of a cream

| Conditions | 6-Phosphogluconate dehydrogenase activity (munit/mg protein) |
|---|---|
| Control | 20.70±0.36 (8) |
| Sunburn | 13.77±1.04 (5) |
| Trifluoperazine | 20.30±0.97 (6) |

Values are means ±S.E., with number of experiments in parentheses. P values (sunburn vs. control or trifluoperazine vs. sunburn)<0.005.

### TABLE 21
Effect of sunburn on the activity of soluble and mitochondrial
hexokinase in rat skin and its reversal by trifluoperazine
administered topically in the form of a cream

| Conditions | Mitochondrial hexokinase activity (munit/mg protein) | Soluble hexokinase activity (munit/mg protein) |
|---|---|---|
| Control | 3.40±0.17 (9) | 2.33±0.09 (9) |
| Sunburn | 1.38±0.08 (5) | 1.18±0.09 (5) |
| Trifluoperazine | 2.19±0.24 (9) | 2.46±0.22 (9) |

Values are means ±S.E., with a number of experiments in parentheses. P values for soluble hexokinase (sunburn vs. control or trifluoperazine vs. sunburn) 0.005; for mitochondrial hexokinase (sunburn vs. control)<0.005; trifluoperazine vs. sunburn)<0.025.

The compound fluphenazine was used to determine its effectiveness in treating experimentally induced burns (100°C water) on rat skin:

### TABLE 22

| Conditions | Hemoglobin (absorption at 420 mµ) |
|---|---|
| Control | 0.20±0.02 (8) |
| Burn | 0.44±0.05 (6) |
| Fluphenazine | 0.32±0.03 (6) |

Values are means ±S.E. with number of experiments in parentheses; P values: burn vs. control <0.005; fluphenazine vs. burn <0.005.

### TABLE 23
Effect on burns of ATP concentration in rat skin
and its reversal by fluphenazine

| Condition | ATP (µmoles/kg wet wt) |
|---|---|
| Control | 1017±42 (6) |
| Burn | 391±44 (6) |
| Fluphenazine | 680±98 (6) |

Values are means ±S.E. with number of experiments in parentheses. P values: burn vs. control <0.005; fluphenazine vs. burn <0.025.

TABLE 24
Effect of burns on the activity of soluble 6-phosphogluconate
dehydrogenase and its reversal by fluphenazine

| Condition | 6-Phosphogluconate dehydrogenase activity (munit/mg protein) |
|---|---|
| Control | 18.37±0.421 (6) |
| Burn | 6.92±0.323 (6) |
| Fluphenazine | 9.69±1.220 (6) |

Values are means ±S.E. with number of experiments in parentheses. P values: burn vs. control <0.005; fluphenazine vs. burn <0.05.

TABLE 25
Effect of burns on the activity of soluble and
mitochondrial hexokinase in rat skin and its
reversal by fluphenazine

| Conditions | Mitochondrial hexokinase activity (munit/mg protein) | Soluble hexokinase activity (munit/mg protein) |
|---|---|---|
| Control | 2.85±0.134 (6) | 2.09±0.088 (6) |
| Burn | 0.78±0.067 (6) | 0.67±0.042 (6) |
| Fluphenazine | 1.48±0.110 (6) | 1.07±0.082 (6) |

Values are means ±S.E., with number of experiments in parentheses. P values (burn vs. control or fluphenazine vs. burn) <0.005.

TABLE 26
Effect of burns on hemoglobin content in rat skin
and its reversal by haloperidol

| Conditions | Hemoglobin (absorption at 420 mµ) |
|---|---|
| Control | 0.20±0.01 (6) |
| Burn | 0.44±0.02 (6) |
| Haloperidol | 0.26±0.02 (6) |

Values are means ±S.E., with number of experiments in parentheses. P values: burn vs. control <0.005; Haloperidol vs. burn <0.005.

TABLE 27
Effect of burns on ATP concentration in rat skin
and its reversal by Haloperidol

| Conditions | ATP (µmoles/kg wet wt) |
|---|---|
| Control | 1151±25 (6) |
| Burn | 322±19 (6) |
| Haloperidol | 645±49 (6) |

Values are means ±S.E., with number of experiments of parentheses. P values: burn vs. control <0.005; Haloperidol vs. burn <0.005.

TABLE 28

Effect of pimozide on hemoglobin content in rat sunburned skin

| Conditions | Hemoglobin (absorption at 420 mμm) |
| --- | --- |
| Control | 0.144±0.015 (16) |
| Sunburn | 0.438±0.043 (7) |
| Pimozide | 0.363±0.034 (11) |

Values are means ±S.E., with number of experiments in parentheses. P values: sunburn vs. control <0.005; pimozide vs. sunburn <0.05.

TABLE 29

Effect of pimozide on ATP concentration in rat sunburned skin

| Conditions | ATP (μmoles/kg wet wt) |
| --- | --- |
| Control | 1231±59 (10) |
| Sunburn | 360±22 (7) |
| Pimozide | 428±22 (11) |

Values are means ±S.E., with number of experiments in parentheses. P values: sunburn vs. control 0.005; pimozide vs. sunburn <0.05.

TABLE 30

Effect of penfluridol on ATP concentration in rat burned skin

| Conditions | ATP (μmoles/kg wet wt) |
| --- | --- |
| Control | 1127±34 (6) |
| Burn | 346±34 (6) |
| Penfluridol | 436±33 (5) |

Values are means ±S.E., with number of experiments in parentheses. P values: burn vs. control 0.005; penfluridol vs. burn <0.05.

TABLE 31

Effect of penfluridol on hemoglobin content in rat burned skin

| Conditions | Hemoglobin (absorption at 420 mμ) |
| --- | --- |
| Control | 0.19±0.02 (6) |
| Burn | 0.53±0.04 (6) |
| Penfluridol | 0.42±0.02 (5) |

Values are means ±S.E., with number of experiments in parentheses. P value: burn vs. control 0.005; penfluridol vs. burn <0.05.

In the experiments demonstrated in Tables 32—43 the compound thioridazine was used to determine its effectiveness in treating experimentally induced burns, sunburns and frostbite. As shown in Tables 32—35 thioridazine reversed the marked changes in the skin content of hemoglobin, ATP and enzymes' activities induced by burns (100°C water). It also reversed the changes in these biochemical parameters induced by sunburns, when administered topically to the sunburned skin in the form of a cream (Tables 36—39). As shown in Tables 40—43, thioridazine was also very effective in the treatment of frostbite. It also has a clearly visible effect on the skin.

TABLE 32
Reversal of the effect of burns (at 100°C)
on hemoglobin content in rat skin by
injection of thioridazine

| Conditions | Hemoglobin (absorption of 420 mµ) |
| --- | --- |
| Control | 0.17±0.02 (8) |
| Burn | 0.42±0.04 (7) |
| Thioridazine | 0.19±0.02 (11) |

Rats (20—25 g b. wt.) were injected i.p. with 2 mg of thiorizadine HCl dissolved in 0.1 ml water, 100 minutes prior to inducing burns. Skin burns were induced by water at 100°C. Twenty minutes later the skins were removed and hemoglobin was extracted and assayed, as described in Methods. Values are means ±S.E., with number of experiments in parentheses. P values (burn vs. control or thioridazine vs. burn) 0.005.

TABLE 33
Reversal of the effect of burns (at 100°C)
on ATP concentration in rat skin by
injection of thioridazine

| Conditions | ATP (µ moles/kg wet wt) |
| --- | --- |
| Control | 1126±60 (6) |
| Burn | 458±28 (6) |
| Thioridazine | 905±79 (9) |

Thioridazine was administered as an injectable solution as described in Table 32. ATP was extracted and assayed, as described in Methods. Values are means ±S.E., with number of experiments in parentheses. P values (burn vs. control or thiorazine vs. burn) <0.005.

TABLE 34
Reversal of the effect of burns (at 100°C) on the activity
of soluble 6-phosphogluconate dehydrogenase in
rat skin by injection of thioridazine

| Conditions | 6-Phosphogluconate dehydrogenase activity (m unit/mg protein) |
| --- | --- |
| Control | 19.23±0.50 (6) |
| Burn | 6.82±0.43 (7) |
| Thioridazine | 15.12±0.48 (6) |

Thioridazine was administered as an injectable solution as described in Table 32. Enzyme was extracted and assayed, as described in Methods. Values are means ±S.E., with number of experiments in parentheses. P values (burn vs. control or thioridazine vs. burn) <0.005.

TABLE 35
Reversal of the effect of burns (at 100°C) on the activity
of soluble and mitochondrial hexokinase in rat skin
by injection of thioridazine

| Conditions | Mitachondrial hexokinase activity (m unit/mg protein) | Soluble hexokinase activity (m unit/mg protein) |
|---|---|---|
| Control | 2.53±0.10 (6) | 1.67±0.10 (6) |
| Burn | 0.72±0.05 (6) | 0.60±0.02 (6) |
| Thioridazine | 1.67±0.21 (6) | 1.54±0.17 (6) |

Thioridazine was administered as an injectable solution as described in Table 32. Enzyme was extracted and assayed, as described in Methods. Values are means ±S.E., with number of experiments in parentheses. P values (burn vs. control or thioridazine vs. burn) <0.005.

TABLE 36
Effect of sunburn on hemoglobin content in rat skin
and its reversal by thioridazine administered
topically in the form of a cream

| Conditions | Hemoglobin (absorption at 420 mμ) |
|---|---|
| Control | 0.16±0.02 (10) |
| Sunburn | 0.48±0.04 (6) |
| Thioridazine | 0.14±0.03 (6) |

400 mg of thioridazine HCl were mixed with 5 g of "Kamill Gesichtscreme" (commercial cosmetic moisture cream). This mixed cream was administered topically to rats (20—25 g b. wt.) two hours prior to the induction of sunburn by a UV lamp (four applications every 30 minutes). It was reapplied immediately following the induction of sunburns and 30 minutes later. After additional 30 minutes, the skin was removed and hemoglobin extracted and assayed, as described in Methods. Values are means ±S.E. with number of experiments in parentheses. P values (sunburn vs. control or thioridazine vs. sunburn) <0.005.

TABLE 37
Effects of sunburn on ATP concentration in rat skin
and its reversal by thioridazine administered
topically in the form of a cream

| Conditions | ATP (μ moles/kg wet wt) |
|---|---|
| Control | 1300±45 (6) |
| Sunburn | 493±37 (6) |
| Thioridazine | 1322±64 (5) |

Thioridazine was administered topically in the form of a cream as described in Table 36. Values are means ±S.E., with number of experiments in parentheses. P values (sunburn vs. control or thioridazine vs. sunburn) <0.005.

18

TABLE 38

Effect of sunburn on the activity of soluble 6-phosphogluconate
dehydrogenase in rat skin and its reversal by thioridazine
administered topically in the form of a cream

| Conditions | 6-Phosphogluconate dehydrogenase activity (m unit/mg protein) |
|---|---|
| Control | 18.65±0.77 (6) |
| Sunburn | 10.10±0.82 (6) |
| Thioridazine | 13.80±0.28 (6) |

Thioridazine was administered topically in the form of a cream as described in Table 36. Values are means ±S.E., with number of experiments in parentheses. P values (sunburn vs. control or thioridazine vs. sunburn) <0.005.

TABLE 39

Effect of sunburn on the activity of soluble hexokinase in
rat skin and its reversal by thioridazine administered
topically in the form of a cream

| Conditions | Soluble hexokinase activity (m unit/mg protein) |
|---|---|
| Control | 2.11±0.09 (7) |
| Sunburn | 0.80±0.10 (6) |
| Thioridazine | 1.96±0.13 (6) |

Thioridazine was administered topically in the form of a cream as described in Table 36. Values are means ±S.E., with number of experiments in parentheses. P values (sunburn vs. control or thioridazine vs. sunburn) 0.005.

TABLE 40

Effect of frostbite on hemoglobin content in rat skin
and its reversal by thioridazine administered
topically in the form of a cream

| Conditions | Hemoglobin (absorption at 420 m$\mu$) |
|---|---|
| Control | 0.18±0.01 (6) |
| Frostbite | 0.36±0.02 (7) |
| Thioridazine | 0.23±0.01 (5) |

Frostbite was induced in the rats under anesthesia by applying dry ice for 1 minute to the skin in the abdomen area. Thioridazine was administered topically in the form of a cream (1 g of thioridazine HCl mixed with 5 g of "Kamill Gesichtrscreme"), immediately following the induction of frostbite, and reapplied 30 minutes later, and then every hour. After 5-1/2 hours from the induction of frostbite, a section of the affected skin was surgically removed and hemoglobin extracted and assayed, as described in Methods. Values are means ±S.E., with number of experiments in parentheses. P values (frostbite vs. control or thioridazine vs. frostbite) <0.005.

# EP 0 168 626 B1

### TABLE 41
#### Effect of frostbite on ATP concentration in rat skin and its reversal by thioridazine administered topically in the form of a cream

| Conditions | ATP (μ moles/kg wet wt) |
|---|---|
| Control | 1063±27 (6) |
| Frostbite | 376±54 (6) |
| Thioridazine | 1003±49 (5) |

Thioridazine was administered topically, as described in Table 40. ATP was extracted and assayed as described in Methods. Values are means S±S.E., with number of experiments in parentheses. P values (frostbite vs. control or thioridazine vs. frostbite) <0.005.

### TABLE 42
#### Effect of frostbite on the activity of soluble 6-phosphoglucomate dehydrogenase in rat skin and its reversal by thioridazine administered topically in the form of a cream

| Conditions | 6-Phosphogluconate dehydrogenase activity (m unit/mg protein) |
|---|---|
| Control | 16.85±0.43 (7) |
| Frostbite | 4.84±0.29 (6) |
| Thioridazine | 13.69±0.46 (5) |

Thioridazine was administered topically as described in Table 40. Enzyme was extracted and assayed, as described in Method. Values are means ±S.E., with number of experiments in parentheses. P values (frostbite vs. control or thioridazine vs. frostbite) <0.005.

### TABLE 43
#### Effect of frostbite on the activity of soluble and mitochondrial hexokinase in rat skin and its reversal by thioridazine administered topically in the form of a cream

| Conditions | Mitochondrial hexokinase activity (m unit/mg protein) | Soluble hexokinase activity (m unit/mg protein) |
|---|---|---|
| Control | 2.63±0.11 (7) | 2.13±0.02 (7) |
| Frostbite | 1.26±0.07 (6) | 0.85±0.04 (6) |
| Thioridazine | 1.84±0.18 (5) | 2.23±0.1 (5) |

Thioridazine was administered topically as described in Table 40. Hexokinase was extracted and assayed, as described in Methods. Values are means ±S.E., with number of experiments in parentheses. P values (frostbite vs. control or thioridazine vs. frostbite) <0.005.

The following compositions may be utilized in the practice of the invention:

Topical ointment

| | |
|---|---|
| Trifluoperazine | 8.0 g |
| Liquid petrolatum | 5.0 g |
| White petrolium | 87.0 g |
| | 100.0 g |

Topical cream

| | |
|---|---|
| Trifluoperazine | 8.0 g |
| Cetyl alcohol | 8.4 g |
| Stearyl alcohol | 8.4 g |
| Sodium lauryl sulfate | 1.4 g |
| White petrolatum | 27.6 g |
| Propylene glycol | 9.2 g |
| Water, to make | 100.0 g |

Topical lotion

| | |
|---|---|
| a. Trifluoperazine | 8.0 g |
| Oil of Olay | 92.0 g |
| | 100.0 g |

| | |
|---|---|
| b. Trifluoperazine | 8.0 g |
| Base* | 92.0 g |
| | 100.0 g |

| | |
|---|---|
| *Stearic acid | 1.4 g |
| Triethanolamine | 0.6 g |
| Glyceryl monostearate | 4.0 g |
| Lanolin, hydrous | 1.0 g |
| Cetyl alcohol | 0.4 g |
| Mineral oil | 2.0 g |
| Methylparahydroxybenzoate | 0.1 g |
| Distilled water | 90.5 g |
| Perfume q.s. | |
| | 100.0 g |

Sunburn preventative

| | |
|---|---|
| Trifluoperazine | 8.0 g |
| Para hydroxybenzoate | 3.0 g |
| Base* | 92.0 g |
| | 100.0 g |

* Topical lotion (b).

Sunburn treatment

| | |
|---|---|
| Trifluoperazine | 8.0 g |
| Lidocaine | 2.0 g |
| Base* | 92.0 g |
| | 100.0 g |

* Topical lotion base (b).

# EP 0 168 626 B1

## Antinfection cream

| | |
|---|---|
| Trifluoperazine | 8.0 g |
| Bacitracin | 40,000 units |
| Cetyl alcohol | 8.4 g |
| Stearyl alcohol | 8.4 g |
| Sodium lauryl sulfate | 1.4 g |
| White petrolatum | 27.6 g |
| Propylene glycol | 9.2 g |
| Water, to make | 100.0 g |

## Anesthetic/antinfection cream

| | |
|---|---|
| Trifluoperazine | 7.0 g |
| Bacitracin | 40,000 units |
| Lidocaine | 1.0 g |
| Cetyl alcohol | 8.4 g |
| Stearyl alcohol | 8.4 g |
| Sodium lauryl sulfate | 1.4 g |
| White petrolatum | 27.6 g |
| Propylene glycol | 9.2 g |
| Water, to make | 100.0 g |

## Spray

| | |
|---|---|
| Trifluoperazine | 8.0 g |
| Propylene glycol | 92.0 g |

## Injectable solution

| | |
|---|---|
| Trifluoperazine | 2 mg |
| Ringer lactate solvent | 1000 ml |

## Flash burn preventive cream

| | |
|---|---|
| Trifluoperazine | 8.0 g |
| Bleached dewaxed shellac | 13.7 g |
| Isopropyl alcohol, 99% | 28.48 g |
| Linseed oil, Z-3 viscosity | 3.5 g |
| Stearic acid, triple pressed | 0.15 g |
| Triethylene glycol di-2-ethylhexoate | 0.8 g |
| Diethylene glycol monoethyl ether | 1.1 g |
| Titanium dioxide | 37 g |
| Sodium bicarbonate | 2.25 g |
| Magnesium stearate | 8 g |
| Menthyl salicylate | 2.5 g |
| Wetting agent (sulfonated alcohol) | 0.3 g |
| Iron oxide (lemon shade) | 1.6 g |
| Mineral black | 0.62 g |

## Claims

1. Use of a composition comprising an ointment, cream or lotion which contains from 4% to 40% by weight of a compound that has the ability to interfere with the action of calcium calmodulin complex in the preparation of a medicament for the therapeutic treatment of trauma to the skin.

2. Use of a composition as defined in Claim 1 wherein the ointment cream or lotion contains from 7.5% to 20% by weight of the compound having the ability to interfere with the action of calcium calmodulin complex.

3. Use of a composition as defined in Claim 1 wherein said compound is selected from the group consisting of phenothiazine, thioxanthenes, butyrophenones, diphenylbutylamines, dibenzodiazepines, benzodiazepines, dibenzazepines and naphthalenesulfonamides that have the ability to interfere with the action of calcium calmodulin complex.

4. Use of a composition as defined in Claim 3 wherein the compound is thioridazine.

5. Use of a composition as defined in Claim 3 wherein the compound is trifluoperazine.

6. Use of a composition in the preparation of a medicament for the therapeutic treatment of burns, sunburn or frostbite said composition comprising an effective amount of a local anesthetic and an amount

22

of a compound having the ability to interfere with the action of calcium calmodulin complex in an ointment, cream or lotion.

7. Use of a composition as defined in Claim 6 wherein the compound is selected from the group consisting of phenothiazines, thioxanthenes, butyrophenones, diphenylbutylamines, dibenzodiazepines, benzodiazepines, dibenzazepines and naphthalenesulfonamides having the ability to interfere with the action of calcium calmodulin complex.

8. Use of a composition as defined in Claim 7 wherein the compound having the ability to interfere with the action of calcium calmodulin complex is trifluoperazine or thioridazine.

9. Use of a composition as defined in Claim 1 which includes an anti-infective agent.

10. Use of a composition as defined in Claim 9 wherein the anti-infective agent is selected from the group consisting of neomycin, bacitracin, silver sulfadiazine, gentamicin, polymyxin and mafenide acetate.

11. Use of a composition as defined in Claim 9 and 10 wherein the compound having the ability to interfere with the action of calcium calmodulin complex is trifluoperazine or thioridazine.

12. Use of a composition as defined in Claim 11 which includes an effective amount of a local anesthetic.

13. Use of a composition comprising an intravenous electrolyte solution and an effective amount of a compound having the ability to interfere with the action of calcium calmodulin complex in the preparation of a medicament for the therapeutic treatment of a patient suffering from a burn, sunburn or frostbite.

14. Use of a composition as defined in Claim 13 wherein the compound having the ability to interfere with the action of calcium calmodulin complex is trifluoperazine or thioridazine.

15. Use of a compound having the ability to interfere with the action of calcium calmodulin complex for the manufacture of a medicament for the therapeutic treatment of burns to the skin.

16. A use as defined in Claim 15, wherein the compound is selected from the group consisting of phenothiazine, thioxanthenes, butyrophenones, diphenylbutylamines, dibenzodiazepines, benzodiazepines, dibenzazepines and naphthalenesulfonamides.

17. A use as defined in Claim 15, wherein the compound is thioridazine.

18. A use as defined in Claim 15, wherein the compound is trifluoperazine.

19. A bandage for application to skin trauma, said bandage comprising in combination an impervious backing layer and an absorbent layer containing a composition which comprises a compound having the ability to interfere with the action of calcium calmodulin complex.

20. A bandage as defined in Claim 19 wherein the compound is trifluoperazine or thioridazine.

## Patentansprüche

1. Verwendung einer Zusammensetzung, die umfaßt eine Salbe, eine Creme oder eine Lotion, die 4 bis 40 Gew.-% einer Verbindung enthält, welche die Fähigkeit hat, die Wirkung des Calcium-Calmodulin-Komplexes zu inhibieren, bei der Herstellung eines Arzneimittels für die therapeutische Behandlung einer Verletzung der Haut.

2. Verwendung einer Zusammensetzung nach Anspruch 1, bei der die Salbe, Creme oder Lotion 7,5 bis 20 Gew.-% der Verbindung enthält, welche die Fähigkeit hat, die Wirkung des Calcium-Calmodulin-Komplxes zu inhibieren.

3. Verwendung einer Zusammensetzung nach Anspruch 1, bei der die Verbindung ausgewählt wird aus der Gruppe, die besteht aus Phenothiazinen, Thioxanthenen, Butyrophenonen, Diphenylbutylaminen, Dibenzodiazepinen, Benzodiazepinen, Dibenzazepinen und Naphtalinsulfonamiden, welche die Fähigkeit haben, die Wirkung des Calcium-Calmodulin-Komplexes zu inhibieren.

4. Verwendung einer Zusammensetzung nach Anspruch 3, bei der die Verbindung Thioridazin ist.

5. Verwendung einer Zusammensetzung nach Anspruch 3, bei der die Verbindung Trifluoperazin ist.

6. Verwendung einer Zusammensetzung bei der Herstellung eines Arzneimittels für die therapeutische Behandlung von Verbrennungen, Sonnenbrand oder Erfrierungen, wobei die Zusammensetzung eine wirksame Menge eines Lokalanästhetikums und eine Menge einer Verbindung enthält, welche die Fähigkeit hat, die Wirkung des Calcium-Calmodulin-Komplexes zu inhibieren, in einer Salbe, Creme oder Lotion.

7. Verwendung einer Zusammensetzung nach Anspruch 6, bei der die Verbindung ausgewählt wird aus der Gruppe, die besteht aus Phenothiazinen, Thioxanthenen, Butyrophenonen, Diphenylbutylaminen, Dibenzodiazepinen, Benzodiazepinen, Dibenzazepinen und Naphtalinsulfonamiden, welche die Fähigkeit haben, die Wirkung des Calcium-Calmodulin-Komplexes zu inhibieren.

8. Verwendung einer Zusammensetzung nach Anspruch 7, bei der die Verbindung, welche die Fähigkeit hat, die Wirkung des Calcium-Calmodulin-Komplexes zu inhibieren, Trifluoperazin oder Thioridazin ist.

9. Verwendung einer Zusammensetzung nach Anspruch 1, die ein Antiinfektionsmittel enthält.

10. Verwendung einer Zusammensetzung nach Anspruch 9, bei der das Antiinfektionsmittel ausgewählt wird aus der Gruppe, die besteht aus Neomycin, Bacitracin, Silbersulfadiazin, Gentamicin, Polymyxin und Mafenidacetal.

11. Verwendung einer Zusammensetzung nach Anspruch 9 und 10, bei der die Verbindung, welche die Fähigkeit hat, die Wirkung des Calcium-Calmodulin-Komplexes zu hemmen, Trifluoperazin oder Thioridazin ist.

12. Verwendung einer Zusammensetzung nach Anspruch 11, die eine wirksame Menge eines Lokalanästhetikums enthält.

13. Verwendung einer Zusammensetzung, die enthält eine intravenöse Elektrolytlösung und eine wirksame Menge einer Verbindung, welche die Fähigkeit hat, die Wirkung des Calcium-Calmodulin-Komplexes zu inhibieren, bei der Herstellung eines Arzneimittels für die therapeutische Behandlung eines Patienten, der an einer Verbrennung, einem Sonnenbrand oder an einer Erfrierung leidet.

14. Verwendung einer Zusammensetzung nach Anspruch 13, bei der die Verbindung, welche die Fähigkeit hat, die Wirkung des Calcium-Calmodulin-Komplexes zu inhibieren, Trifluoperazin oder Thioridazin ist.

15. Verwendung einer Verbindung, welche die Fähigkeit hat, die Wirkung des Calcium-Calmodulin-Komplexes zu inhibieren, für die Herstellung eines Arzneimittels für die therapeutische Behandlung von Hautverbrennungen.

16. Verwendung nach Anspruch 15, bei der die Verbindung ausgewählt wird aus der Gruppe, die besteht aus Phenothiazinen, Thioxanthenen, Butyrophenonen, Diphenylbutylaminen, Dibenzodiazepinen, Benzodiazepinen, Dibenzazepinen und Naphtalinsulfonamiden.

17. Verwendung nach Anspruch 15, bei der die Verbindung Thioridazin ist.

18. Verwendung nach Anspruch 15, bei der die Verbindung Trifluoperazin ist.

19. Bandage (Verband) zum Aufbringen auf eine Hautverletzung, die (der) umfaßt eine undurchlässige Unterlagenschicht in Kombination mit einer Adsorbens-Schicht, enthaltend eine Zusammensetzung, die eine Verbindung enthält, welche die Fähigkeit hat, die Wirkung des Calcium-Calmodulin-Komplexes zu inhibieren.

20. Bandage (Verband) nach Anspruch 19, bei der (dem) die Verbindung Trifluoperazin oder Thioridazin ist.

**Revendications**

1. Utilisation d'une composition comprenant une pommade, une crème ou une lotion, qui contient de 4 à 40% en poids d'un composé qui est capable d'interférer avec l'action du complexe calmoduline de calcium, dans la préparation d'un médicament pour le traitement thérapeutique d'un trauma de la peau.

2. Utilisation d'une composition selon la revendication 1, dans laquelle la pommade, crème ou lotion contient de 7,5% à 20% en poids du composé qui est capable d'interférer avec l'action du complexe calmoduline de calcium.

3. Utilisation d'une composition selon la revendication 1, dans laquelle ledit composé est choisi parmi la phénothiazine, les thioxanthènes, les butyrophénones, les diphénylbutylamines les dibenzodiazépines, les benzodiazépines, les dibenzazépines et les naphtalènesulfonamides qui sont capables d'interférer avec l'action du complexe calmoduline de calcium.

4. Utilisation d'une composition selon la revendication 3, dans laquelle le composé est la thioridazine.

5. Utilisation d'une composition selon la revendication 3, dans laquelle le composé est la trifluopérazine.

6. Utilisation d'une composition dans la préparation d'un médicament pour le traitement thérapeutique des brûlures, des coups de soleil ou des gelures, ladite composition comprenant une quantité efficace d'un anesthésique local et une quantité d'un composé capable d'interférer avec l'action du complexe calmoduline de calcium dans une pommade, une crème ou une lotion.

7. Utilisation d'une composition selon la revendication 6, dans laquelle le composé est choisi parmi les phénothiazines, les thioxanthènes, les butyrophénones, les diphénylbutylamines, les dibenzodiazépines, les benzodiazépines, les benzodiazépines, les dibenzazépines et les naphtalènesulfonamides capables d'interférer avec l'action du complexe calmoduline de calcium.

8. Utilisation d'une composition selon la revendication 7, dans laquelle le composé capable d'interférer avec l'action du complexe calmoduline de calcium est la trifluopérazine ou la thioridazine.

9. Utilisation d'une composition selon la revendication 1 qui contient un agent anti-infectieux.

10. Utilisation d'une composition selon la revendication 9, dans laquelle l'agent anti-infectieux est choisi parmi la néomycine, la bacitracine, la sulfadiazine d'argent, la gentamicine, la polymyxine et l'acétate de mafénide.

11. Utilisation d'une composition selon les revendications 9 et 10, dans laquelle le composé capable d'interférer avec l'action du complexe calmoduline de calcium est la trifluopérazine ou la thioridazine.

12. Utilisation d'une composition selon la revendication 11 qui contient une quantité efficace d'un anesthésique local.

13. Utilisation d'une composition comprenant une solution intraveineuse d'électrolyte et une quantité efficace d'un composé capable d'interférer avec l'action du complexe calmoduline de calcium dans la préparation d'un médicament pour le traitement thérapeutique d'un patient souffrant d'une brûlure, d'un coup de soleil ou d'une gelure.

14. Utilisation d'une composition selon la revendication 13, dans laquelle le composé capable d'interférer avec l'action du complexe calmoduline de calcium est la trifluopérazine ou la thioridazine.

15. Utilisation d'un composé capable d'interférer avec l'action du complexe calmoduline de calcium pour la fabrication d'un médicament pour le traitement thérapeutique des brûlures de la peau.

16. Utilisation selon la revendication 15, dans laquelle le composé est choisi parmi la phénothiazine, les thioxanthènes, les butyrophénones, les diphénylbutylamines, les dibenzodiazépines, les benzodiazépines, les dibenzazépines et les naphtalènesulfonamides.

17. Utilisation selon la revendication 15, dans laquelle le composé est la thioridazine.

18. Utilisation selon la revendication 15, dans laquelle le composé est la trifluopérazine.

19. Un bandage pour l'application à un traumatisme de la peau, ledit bandage comprenant en combinaison une couche dorsale imperméable et une couche absorbante contenant une composition qui comprend un composé capable d'interférer avec l'action du complexe calmoduline de calcium.

20. Un bandage selon la revendication 19, dans lequel le composé est la trifluopérazine ou la thioridazine.

Fig. 1

HEMOGLOBIN
(Absorption at 420 mµ)

A.

Control (8)

Burn (9)

TRiFLUOPERAZINE (6)

B.

Control (8)

Burn (9)

TRIFLUOPERAZINE (8)